# EUROPÄISCHE PATENTSCHRIFT

(11) **EP 0 946 489 B1**
(45) Veröffentlichungstag und Bekanntmachung des Hinweises auf die Patenterteilung: **16.08.2001**
(21) Anmeldenummer: 97953727.1
(22) Anmeldetag: 01.12.1997
(51) Int. Cl.: C07C 67/26, C07C 69/28, C07C 69/52

(54) **VERFAHREN ZUR HERSTELLUNG VON FETTSÄUREPOLYETHYLENGLYCOLESTERN**
PROCESS FOR PREPARING FATTY ACID POLYETHYLENE GLYCOL ESTERS
PROCEDE DE PREPARATION D'ESTERS DE POLYETHYLENEGLYCOL D'ACIDE GRAS

(30) Priorität: 09.12.1996 US 767123
(43) Veröffentlichungstag der Anmeldung: 06.10.1999
(73) Patentinhaber: Cognis Deutschland GmbH, 40589 Düsseldorf (DE)
(72) Erfinder: RATHS, Hans-Christian, D-40789 Monheim (DE); BEHLER, Ansgar, D-46240 Bottrop (DE); MILSTEIN, Norman, Montgomery, OH 45242 (US)
(86) Internationale Anmeldenummer: EP9706705
(87) Internationale Veröffentlichungsnummer: WO9825878

(56) Entgegenhaltungen:
- EP-A- 0 178 913

## Beschreibung

Die Anmeldung betrifft ein Verfahren zur Herstellung von Fettsäurepolyethylenglycolestem durch Ethoxylierung von Fettsäuren in Gegenwart von Alkanolaminen.

### Stand der Technik

Die Anlagerung von Alkylenoxiden an CH-acide Verbindungen wie beispielsweise Fettalkohole, Alkylphenole, Fettamine oder auch Fettsäuren zählt zu den großtechnisch etablierten Verfahren zur Herstellung von nichtionischen Tensiden. Üblicherweise werden diese Reaktionen in Gegenwart homogener basischer Katalysatoren wie beispielsweise Natriumhydroxid oder Natriummethylat durchgeführt. Die Alkoxylierung stellt jedoch eine wenig selektive Reaktion dar, die vielmehr der Statistik folgt. Demzufolge läßt sich aus dem molekularen Einsatzverhältnis von CH-acider Verbindung und Alkylenoxid bestenfalls auf den durchschnittlichen Alkoxylierungsgrad schließen. In der Praxis beobachtet man jedoch, daß insbesondere bei niedrigen Alkoxylierungsverhältnissen das Maximum der resultierenden Homologenverteilung nicht einmal beim durchschnittlichen Alkoxylierungsgrad zu finden, sondern in der Regel zu höheren Homologen verschoben ist.

Man versucht diesem unerwünschten Effekt zu begegnen, indem man Katalysatoren einsetzt, die eine höhere Selektivität aufweisen und in Summe zu Alkoxylaten, speziell Ethoxylaten, mit engerer ("eingeengter") Homologenverteilung führen; diese Produkte werden in der Literatur häufig auch als "narrow-range ethoxylates" bezeichnet. Als homogene Katalysatoren kommen für diesen Zweck vorzugsweise Erdalkalisalze, wie beispielsweise Bariumphosphat oder Strontiumethercarboxylate in Frage. Auch heterogene Katalysatoren wie beispielsweise calcinierte Hydrotalcite eignen sich hierfür.

Bezüglich der Ethoxylierung von Fettsäuren sind die Bemühungen nach dem Stand der Technik indes nach wie vor nicht befriedigend. Insbesondere wenn es darum geht, niedrig ethoxylierte Fettsäuren, speziell Fettsäure+1EO-Addukte, herzustellen, die als Vorstufen zur Synthese von Ethersulfat-Tensiden mit isethionatartiger Struktur interessant sind, werden nicht befriedigende Selektivitäten festgestellt. Neben einem unerwünschten Anteil höher ethoxylierter Homologer werden dabei insbesondere auch signifikante Mengen an Polyethylenglycol und Diestern gebildet. Auch das Verfahren gemäß dem US-Pa-tent **US 3,884,946** (Henkel), das für diesen Zweck die Verwendung von Aminen als Katalysatoren empfiehlt, liefert die niedrig ethoxylierten Fettsäuren in Ausbeuten deutlich unter 90 % der Theorie.

Demzufolge hat die Aufgabe der vorliegenden Erfindung darin bestanden, ein verbessertes homogenchemisches Verfahren zur Herstellung von Fettsäurepolyglycolestem, speziell niedrig ethoxylierten Fettsäuren, zur Verfügung zu stellen, das sich durch eine verbesserte Selektivität auszeichnet.

### Beschreibung der Erfindung

Gegenstand der Erfindung ist ein Verfahren zur Herstellung von Fettsäurepolyethylenglycolestem der Formel (I),

**R**^{**1**}**CO(CH**_{**2**}**CH**_{**2**}**O)**_{**n**}**H** (I)

in der R¹CO für einen linearen oder verzweigten, aliphatischen, gesättigten und/oder ungesättigten Acylrest mit 6 bis 22 Kohlenstoffatomen und n für Zahlen von 0,5 bis 1,5 steht, durch basisch homogen-kataysierte Anlagerung von Ethylenoxid an Fettsäuren, welches sich dadurch auszeichnet, daß man die Ethoxylierung in Gegenwart von Alkanolaminen durchführt.

Überraschenderweise wurde gefunden, daß Alkanolamine, speziell Triethanolamin, außerordentlich selektive Katalysatoren für die Ethoxylierung von Fettsäuren darstellen, insbesondere dann, wenn es darum geht, niedrig ethoxylierte Homologe herzustellen.

### Fettsäuren

Unter Fettsäuren sind aliphatische Carbonsäuren der Formel (II) zu verstehen,

**R**^{**1**}**COOH** (II)

in der R¹CO für einen aliphatischen, linearen oder verzweigten Acylrest mit 6 bis 22, vorzugsweise 12 bis 18 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkem- oder Talgfettsäure.

### Alkanolamine

Typische Beispiele für Alkanoiamine, die als basische Katalysatoren in Betracht kommen, sind Monoethanolamin, Diethanolamin und vorzugsweise Triethanolamin. Üblicherweise werden die Alkanolamine in Mengen von 0,1 bis 5, vorzugsweise 0,5 bis 1,5 Gew.-% - bezogen auf die Fettsäuren - eingesetzt.

### Ethoxylierung

Die Ethoxyiierung kann in an sich bekannter Weise durchgeführt werden. Üblicherweise legt man die Fettsäure und den Katalysator in einem Rührautoklaven vor, den man vor der Reaktion durch abwechselndes Evakuieren und Stickstoffspüien von Wasserspuren befreit. Anschließend wird die Fettsäure mit dem Ethylenoxid im molaren Verhältnis 1 : 0,5 bis 1 : 1,5 mit Ethylenoxid umgesetzt, welches man nach dem Aufheizen portionsweise über einen Heber in den Druckbehälter eindosieren kann. Vorzugsweise werden die Fettsäuren mit einem Mol Ethylenoxid umgesetzt. Die Ethoxylierung kann bei Temperaturen im Bereich von 80 bis 180, vorzugsweise 100 bis 120°C und autogenen Drücken im Bereich von 1 bis 5, vorzugsweise 2 bis 3 bar durchgeführt werden. Nach Reaktionsende empfiehlt es sich, zur Vervollständigung des Umsatzes eine gewisse Zeit bei der Reaktionstemperatur nachzurühren (15-90min). Anschließend wird der Autoklav abgekühlt, entspannt und das Produkt, falls dies gewünscht wird, mit Säuren wie z.B. Milchsäure oder Phosphorsäure versetzt, um den basischen Katalysator zu neutralisieren.

### Beispiele

### Beispiel 1:

In einem 1-l-Rührautoklaven wurden 200 g (1 Mol) technische Laurinsäure vorgelegt und mit 2 g Triethanolamin (entsprechend 1 Gew.-% bezogen auf Laurinsäure) versetzt. Der Autoklav wurde dreimal abwechselnd evakuiert und mit Stickstoff beaufschlagt um Spuren von Wasser, die zur Bildung von Polyethylenglycol führen könnten, zu entfernen. Nachdem die Reaktionsmischung ein letztesmal mit Stickstoff beaufschlagt worden war, wurde der Autoklav verschlossen auf 100°C erhitzt und portionsweise bei einem Maximaldruck von 5 bar mit 44 g (1 Mol) Ethylenoxid beaufschlagt. Nach Abschluß der Reaktion, erkenntlich dadurch, daß der Druck wieder bis auf einen Wert von 1,2 bar abfiel und dann konstant blieb, wurde 30 min nachgerührt und der Reaktionsansatz anschließend abgekühlt und entspannt. Der basische Katalysator wurde durch Zusatz einer entsprechenden Menge Milchsäure neutralisiert. Die Kenndaten des Laurinsäure+1EO-Adduktes sind in Tabelle 1 zusammengefaßt.

### Beispiel 2

Beispiel 1 wurde mit 60 min Nachrühren wiederholt. Die Ergebnisse sind in Tabelle 1 zusammengefaßt.

### Beispiele 3 und 4

Beispiel 2 wurde mit (3) 0,5 Gew.-% bzw. (4) 1 Gew.-% Triethanolamin wiederholt. Die Ergebnisse sind in Tabelle 1 zusammengefaßt.

### Beispiele 5 und 6

Beispiel 2 wurde mit (5) 80°C bzw. (6) 90°C wiederholt. Die Ergebnisse sind in Tabelle 1 zusammengefaßt.

### Beispiele 7 bis 9

Beispiel 2 wurde unter Einsatz von jeweils 1 Mol (7) C_{12/14}-Kokosfettsäure, (8) C_{12/18}-Kokosfettsäure bzw. (9) C_{16/18}-Talgfettsäure und 1 Mol Ethylenoxid wiederholt. Die Ergebnisse sind in Tabelle 1 zusammengefaßt.

### Vergleichsbeispiel V1

Beispiel 1 wurde mit 0,5 Gew.% calciniertem Hydrotalcit als Katalysator bei 145-160 °C wiederholt. Die Ergebnisse sind in Tabelle 1 dargestellt.

**Tabelle 1**

| Zusammensetzung der Fettsäure+EO-Addukte (Mengenangaben als Gew.-%) | | | | | | | | | | |
|---|---|---|---|---|---|---|---|---|---|---|
| | **1** | **2** | **3** | **4** | **5** | **6** | **7** | **8** | **9** | **V** |
| Polyethylenglycolgehalt | <0,1 | <0,1 | <0,1 | <0,1 | <0,1 | <0,1 | <0,1 | <0,1 | <0,1 | 1.1 |
| Freie Fettsäure | 4,3 | 5,0 | 9,9 | 6,4 | 43,3 | 18,0 | 3,8 | 6,4 | 6,7 | 12,0 |
| Fettsäure+1EO-Addukt | 89,0 | 87,3 | 79,6 | 85,0 | 52,1 | 75,5 | 86,4 | 85,0 | 84,2 | 45.0 |
| Fettsäure+2EO-Addukt | 2,7 | 2,8 | 4,0 | 2,1 | 1,9 | 2,8 | 3,0 | 2,1 | 2,7 | 2.0 |
| Fettsäure+3EO-Addukt | 0,1 | <0,1 | <0,1 | <0,1 | <0,1 | <0,1 | <0,1 | <0.1 | <0,1 | 0.3 |
| Diester | 6.3 | 3,8 | 5,0 | 4,3 | 1,1 | 1,7 | 5,6 | 4,3 | 4,6 | 39,2 |
| Höhre Homologen | ad 100 | | | | | | | | | |

## Patentansprüche

1. Verfahren zur Herstellung von Fettsäurepolyglycolestem der Formel (I),
**R**^{**1**}**COO(CH**_{**2**}**CH**_{**2**}**O)**_{**n**}**H** (I)
in der R¹CO für einen linearen, aliphatischen, gesättigten und/oder ungesättigten Acylrest mit 6 bis 22 Kohlenstoffatomen und n für Zahlen von 0,5 bis 1,5 steht, durch basisch homogen-katalysierte Anlagerung von Ethylenoxid an Fettsäuren in Gegenwart von Alkanolaminen.

2. Verfahren nach Anspruch 1, **dadurch gekennzeichnet,** daß man Fettsäuren der Formel (II) einsetzt,
**R**^{**1**}**COOH** (II)
in der R¹CO für einen linearen, aliphatischen, gesättigten Acylrest mit 12 bis 18 Kohlenstoffatomen einsetzt.

3. Verfahren nach den Ansprüchen 1 und 2, **dadurch gekennzeichnet,** daß man die Fettsäuren mit etwa 1 Mol Ethylenoxid umsetzt.

4. Verfahren nach den Ansprüchen 1 bis 3, **dadurch gekennzeichnet**, daß man als basischen Katalysator Triethanolamin einsetzt.

5. Verfahren nach den Ansprüchen 1 bis 4. **dadurch gekennzeichnet**, daß man die Alkanolamine in Mengen von 0,1 bis 5 Gew.-% - bezogen auf die Fettsäuren - einsetzt.

6. Verfahren nach den Ansprüchen 1 bis 5, **dadurch gekennzeichnet,** daß man die Ethoxylierung bei Temperaturen im Bereich von 80 bis 180°C und autogenen Drücken im Bereich von 1 bis 5 bar durchführt.

## Claims

1. A process for the production of fatty acid polyglycol esters corresponding to formula (I):
R¹COO(CH₂CH₂O)ₙH (I)
in which R¹CO is a linear, aliphatic, saturated and/or unsaturated acyl group containing 6 to 22 carbon atoms and n is a number of 0.5 to 1.5, by homogeneously base-catalysed addition of ethylene oxide onto fatty acids in the presence of alkanolamines.

2. A process as claimed in claim 1, characterized in that fatty acids corresponding to formula (II):
R¹COOH (II)
in which R¹CO is a linear, aliphatic saturated acyl group containing 12 to 18 carbon atoms,
are used.

3. A process as claimed in claims 1 and 2, characterized in that the fatty acids are reacted with about 1 mole of ethylene oxide.

4. A process as claimed in claims 1 to 3, characterized in that triethanolamine is used as the basic catalyst.

5. A process as claimed in claims 1 to 4, characterized in that the alkanolamines are used in quantities of 0.1 to 5% by weight, based on the fatty acids.

6. A process as claimed in claims 1 to 5, characterized in that the ethoxylation is carried out at temperatures of 80 to 180°C and under autogenous pressures of 1 to 5 bar.

## Revendications

1. Procédé de préparation d'esters de polyglycol et d'acide gras de formule (I)
R¹CO(CH₂CH₂O)ₙH (I)
dans laquelle R¹CO représente un reste acyle linéaire ou ramifié, aliphatique, saturé et/ou non saturé, ayant de 6 à 22 atornes de carbone et n représente des nombres allant de 0,5 à 1,5, par addition par catalyse homogène en milieu basique d'oxyde d'éthylène sur des acides gras en présence d'alkanolamines.

2. Procédé selon la revendication 1,
caractérisé en ce qu'
on met en oeuvre des acides gras de formule (II),
R¹COOH (II)
dans laquelle R¹CO représente un reste acyle linéaire, aliphatique, saturé ayant de 12 à 18 atomes de carbone.

3. Procédé selon l'une des revendications 1 ou 2,
caractérisé en ce qu'
on fait réagir les acides gras avec environ 1 mol d'oxyde d'éthylène.

4. Procédé selon l'une quelconque des revendications 1 à 3,
caractérisé en ce qu'
on met en oeuvre comme catalyseur basique de la triéthanolamine.

5. Procédé selon l'une quelconque des revendications 1 à 4,
caractérisé en ce qu'
on met en oeuvre les alkanolamines en quantités allant de 0,1 à 5 % en poids rapporté aux acides gras.

6. Procédé selon l'une quelconque des revendications 1 à 5,
caractérisé en ce qu'
on effectue l'éthoxylation à des températures dans la zone allant de 80 à 180°C et à des pressions autogènes dans la zone allant de 1 à 5 bars.
